# EUROPEAN PATENT APPLICATION

(11) **EP 3 486 916 A1**
(43) Date of publication of application: **22.05.2019**
(21) Application number: 17202613.0
(22) Date of filing: 20.11.2017
(51) Int. Cl.: G16H 40/20, G16H 40/40

(54) **METHOD AND SYSTEM FOR LOCALIZING A HANDHELD ANALYTICAL DEVICE**

(71) Applicant: F. Hoffmann-La Roche AG, 4070 Basel (CH); Roche Diagnostics GmbH, 68305 Mannheim (DE)
(72) Inventor: HOFSTETTER, Hermann, 6343 Rotkreuz (CH); KOCHERSCHEIDT, Gerrit, 68305 Mannheim (DE); KORNER, Stephan, 6343 Rotkreuz (CH); PORSCH, Ulrich, 68305 Mannheim (DE); STEINERT, Chris, 6343 Rotkreuz (CH)
(74) Representative: Gyenge, Zoltán

(57) **Abstract**

Computer implemented method for localizing handheld analytical devices (10) in a point of care environment, comprising: associating a location identifier (30) to a plurality of locations within the point of care environment and storing said location identifier (30) into a database (22); at each of the plurality of locations, capturing signal patterns using signal receiver(s) of the handheld analytical devices (10); storing all signal patterns captured in a database (22); capturing a current signal pattern using signal receiver(s) of a handheld analytical device (10) to be localized; the control unit (20) providing a descriptive localization indicative of its position relative to one or more of the plurality of locations within the point of care environment associated with one or more signal pattern(s) stored in the database (22) matching the current signal pattern.

## Description

### TECHNICAL FIELD

The present application relates to a computer implemented method for localizing handheld analytical devices in a point of care environment. The application further relates to a point of care system for analyzing biological samples configured to localize handheld analytical devices as well as a computer program product comprising instructions which, when executed by a computer system, cause a point of care system to localize handheld analytical devices in a point of care environment.

### BACKGROUND

In vitro diagnostic testing has a major effect on clinical decisions, providing physicians with pivotal information. Particularly, there is great emphasis on providing quick and accurate test results in critical care settings.

One particular type of diagnostic testing is bedside testing or point of care POC testing. This type of diagnostic testing is performed mainly by nurses or medical staff primarily trained to operate the instruments available at the site of patient care, such as hospitals, emergency departments, intensive care units, primary care setting, medical centers, patient homes, a physician's office, a pharmacy or a site of an emergency. Major benefits are obtained when the measurement results obtained by point of care testing POC device(s) is made available immediately/results can be shared instantaneously with all members of the medical team, thereby enhancing communication through decreasing turnaround time.

Point of care testing has become established worldwide and finds vital roles in public health. Potential operational benefits of point of care testing include: faster decision making, reduced operating times, reduced postoperative care time, reduced emergency room time, reduced number of outpatient clinic visits, reduced number of hospital beds required and overall optimal use of professional time.

Generally, the goal of Point of Care is to help both healthcare professionals and patients achieve improved clinical and health-economic outcomes, by delivering robust, connected, easy to use point of care solutions outside the central lab, providing immediate results and thus allowing treatment decisions to be made more quickly - inside or outside the hospital. Point of Care testing delivers those solutions meeting the clinical need for quick and accurate test results delivered where needed, when needed; on the device, in an electronic healthcare record on a patient/ward monitor, to the clinician on the move and directly to the patient.

While there are many benefits of using point of care testing devices in terms of their convenience, establishing point of care testing environments poses several challenges as well. Extensive point of care environments such as large hospitals can have an install base of several hundred up to thousands of point of care devices, in particular handheld analytical devices. Sometimes these handheld analytical devices are not brought back to their respective charging units or get misplaced somewhere in the hospital. Hence there is a need for a solution to aid in localizing handheld analytical devices in a point of care environment.

So-called Real-Time Location Systems RTLS are known in the art which, among other features, provide asset tracking in the healthcare sector. RTLS technology relies strongly on dedicated hardware (e.g. RF beacons) and extensive setup (e.g. floorplans, signal mapping/landscaping, teaching activities, etc.) to be used in determining the absolute location of the assets, usually in real-time. However, such Real-Time Location Systems RTLS are associated with significant up-front installation costs and effort as well as high maintenance costs of the additional infrastructure needed.

It shall be noted that localization of the handheld analytical devices is not the main objective in point of care testing - which is the delivery of test results - but rather a support functionality to enable flawless functionality of point of care testing systems. Hence, providers of point of care testing environments are often not willing or capable to support the significant up-front installation costs and effort associated with existing Real-Time Location Systems RTLS.

Therefore, there is a need for a cost-effective solution to localize handheld analytical devices in a point of care environment.

### SUMMARY

The inventors of the present disclosure recognized that often an absolute determination of the actual position of the handheld devices is not necessary in order to aid operators in finding the handheld devices. Instead, in most cases a relative determination of the location of handheld analytical devices is more than sufficient, such as an indication that a particular handheld analytical device is at its docking station, at the calibration station, etc. This is also called soft localization.

Disclosed herein is a computer implemented method for localizing handheld analytical devices in a point of care environment, the point of care environment comprising a plurality of handheld analytical devices for analyzing biological samples communicatively connected to a control unit, the method comprising:
- associating a location identifier corresponding to one or more of a plurality of locations within the point of care environment and storing said location identifier into a database communicatively connected to or comprised by the control unit;
- at each of the plurality of locations, capturing signal patterns using signal receiver(s) of the plurality of the handheld analytical devices;
- storing all signal patterns captured by the plurality of handheld analytical devices in a database, each signal pattern being associated with the location identifier where the signal pattern has been captured;
- capturing a current signal pattern using signal receiver(s) of a handheld analytical device to be localized;
- the control unit providing a descriptive localization, the descriptive localization comprising an indication of a position of the handheld analytical device to be localized relative to one or more of the plurality of locations within the point of care environment associated with one or more signal pattern(s) stored in the database matching the current signal pattern.

Furthermore, the present application relates to a point of care system for analyzing biological samples comprising:
- a plurality of handheld analytical devices for analyzing biological samples;
- a control unit communicatively connected to the plurality of handheld analytical devices by means of a communication network
- a database communicatively connected to or comprised by the control unit;
wherein the point of care system is configured such as to carry out one of the methods according to any one of the embodiments of the disclosed method.

Collective teaching of the signal patterns by the plurality of the handheld analytical devices provides for a significant reduction of set-up costs and efforts, since no dedicated hardware is required for capturing the signal patterns in the various locations of the point of care environment. Furthermore, embodiments of the disclosed method/system enable an iterative capture, respectively storage (in the database) of the signal patterns at the various locations (teaching), each new capture of a signal pattern by any one of the handheld devices expanding the capabilities/accuracy/speed of the localization of all handheld devices. Hence, the invention disclosed provides the option to incrementally build up the database of signal patterns. In this way users/operators of the disclosed method/system can benefit from a low level of up-front effort and can start using the method/system to localize handheld analytical devices as soon as at least one signal pattern is captured.

Embodiments herein disclosed are further advantageous in that by using the handheld analytical devices themselves for capturing the signal patterns (vs. specialized hardware) enables operators thereof to incrementally build up the database of signal patterns during their daily routine. This aspect of the disclosed method/system saves considerable amount of time and also contributes to aligning the descriptive localization of the handheld analytical devices to locations relevant in the context of the daily routine in the point of care environment - as compared to often "synthetic" locations pre-defined in known localization systems, pre-defined locations which often have little relevance to the actual use of the devices. In addition, off-the-shelf portable computing devices (such as smartphones) may also be used to complement the teaching /capture of signal patterns across the point of care environment.

Further embodiments of the disclosed method/system, enrich the descriptive localization of the handheld analytical devices by providing action information (as part of the descriptive localization) related to user action(s) historically carried out at the current location of the searched handheld analytical device.

In order to enable some level of localization even when localization based on current signal pattern would otherwise not be possible, further embodiments herein disclosed rely - in addition to the current signal pattern - on a currently performed action on the handheld analytical device which is to be localized.

Hence - according to particular embodiment(s) - capturing user actions have a double purpose: on one hand action information is used to enrich the descriptive localization; while on the other hand the history of user actions may substitute and/or complement the signal patterns in localizing the handheld analytical device.

### BRIEF DESCRIPTION OF THE DRAWINGS

Further characteristics and advantages of the disclosed method /device /system will in the following be described in detail by means of the description and by making reference to the following drawings:
Fig. 1 a schematic block diagram of a point of care system according to the present disclosure;
Fig. 2 a flowchart illustrating a first embodiment of the method for localizing handheld analytical devices;
Fig. 3 a flowchart illustrating a further embodiment of the method for localizing handheld analytical devices, wherein descriptive localization is enriched with action information;
Fig. 4 a flowchart illustrating an even further embodiment of the method for localizing handheld analytical devices, wherein availability of the localization is extended (to cases where signal pattern based localization would not suffice) by capturing most recent action carried out with the handheld analytical device to be localized;
Fig. 5 a schematic diagram of a point of care system according to the present disclosure as deployed in a point of care environment.

### DETAILED DESCRIPTION

Certain terms will be used in this patent application, the formulation of which should not be interpreted to be limited by the specific term chosen, but as to relate to the general concept behind the specific term.

The terms '**sample**', '**patient sample**' and '**biological sample**' refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The term '**analysis**' or '**analytical test**' as used herein encompasses a laboratory procedure characterizing a parameter of a biological sample, e.g. light absorption, fluorescence, electrical potential or other physical or chemical characteristics of the reaction to provide the measurement data.

The term '**point of care POC**' or '**point of care POC environment**' as used herein is defined to mean a location on or near a site of patient care where medical or medically related services such as medical testing and/or treatment are provided, including but not limited to hospitals, emergency departments, intensive care units, primary care setting, medical centers, patient homes, a physician's office, a pharmacy or a site of an emergency.

The term '**point of care testing POCT**' as used herein encompasses analysis of one or more patient(s) or patient sample(s) for one or more patient health parameters in a point of care POC environment. Point of care testing POCT can be often accomplished through the use of transportable, portable, and handheld analytical devices, but small bench-top analyzers or fixed equipment can also be used when a handheld device is not available- the goal being to collect the patient health parameter and obtain analytical data in a (relatively) short period of time at or (relatively) near the location of the patient.

The term '**analytical device**' as used herein refers to an apparatus configured to obtain a measurement value. An analytical device is operable to determine via various chemical, biological, physical, optical, electro-chemical or other technical procedures a parameter value of the sample or a component thereof. An analytical device may be operable to measure said parameter of the sample or of at least one analyte and return the obtained measurement value. The list of possible analysis results returned by the analytical device comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical device may comprise units assisting with the pipetting, dosing, and mixing of samples and/or reagents. The analytical device may comprise a reagent holding unit for holding reagents to perform the assays.

The term '**user interface**' as used herein encompasses any suitable piece of software and/or hardware for interactions between an operator and a machine, including but not limited to a graphical user interface for receiving as input a command from an operator and also to provide feedback and convey information thereto. Also, a system/device may expose several user interfaces to serve different kinds of users/operators.

The term '**analytical data**' as used herein encompasses any data that is descriptive of a result or partial result of a measurement or processing of a biological sample. In case of a calibration the analytical data comprises the calibration result, i.e. calibration data. In particular, the analytical data comprises an identifier of the sample for which the analysis has been performed and data being descriptive of a result of the analysis, such as measurement data.

Particular embodiments of the disclosed method/system shall be described as follows with reference to the figures.

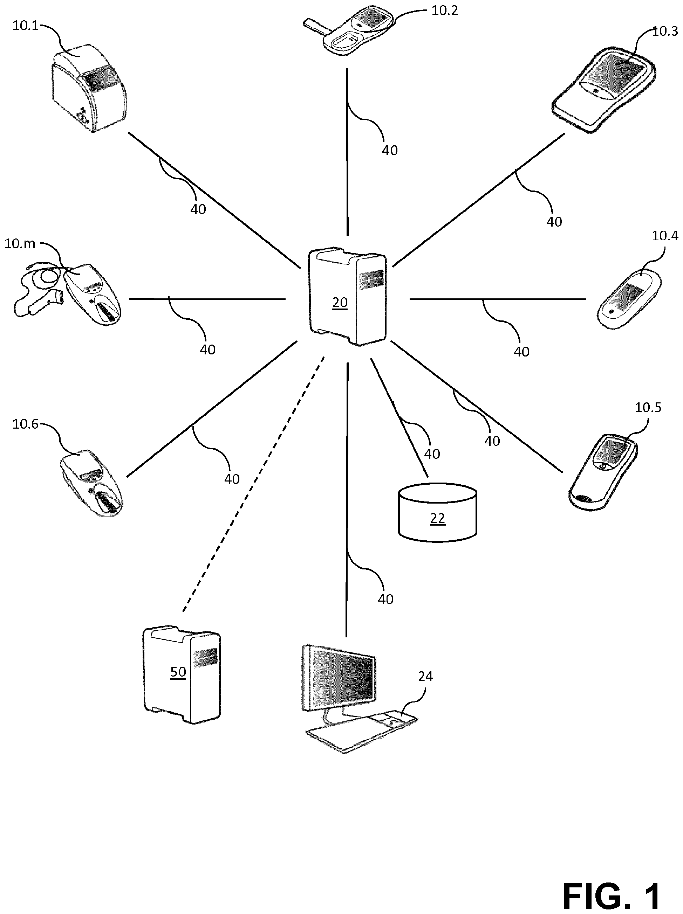

Figure 1 shows a schematic block diagram of a point of care system 1 according to the present disclosure comprising a plurality of handheld analytical devices 10 communicatively connected to a control unit 20 by means of a communication network 40. The term '**handheld analytical device**" 10 as used herein refers to an analytical device used in a point of care POC environment which can be carried/relocated by medical staff usually on the premises of a point of care environment. Handheld analytical devices 10 include devices for (but not limited to) blood glucose testing, coagulation testing, blood gas and electrolytes analysis, urinalysis, cardiac markers analysis, hemoglobin diagnostics, infectious disease testing, cholesterol screening or nucleic acid testing.

The term '**control unit**' 20 as used herein encompasses any physical machine or virtual machine having a physical or virtual processor, capable of accepting requests from and giving responses accordingly. It shall be clear to a person of ordinary skill in the art of computer programming that the term machine may refer to a physical hardware itself, or to a virtual machine such as a JAVA Virtual Machine JVM, or even to separate virtual machines running different Operating Systems on the same physical machine and sharing that machine's computing resources. A control unit can run on any computer including dedicated computers, which individually are also often referred to as a server or shared resources such as virtual servers. In many cases, a computer can provide several services and have several servers running. Therefore, the term control unit shall encompass any computerized device that shares a resource with one or more client processes.

The term '**communication network**' 40 as used herein encompasses any type of wireless network such as a WIFI, GSM, UMTS or other wireless digital network; respectively a cable based network, such as Ethernet or the like. For example, the communication network 40 comprises a combination of wired and wireless networks.

A database 22 is either comprised by or communicatively connected to the control unit 20. The term '**database**' 22 shall be used herein to refer to any kind of storage, including volatile or non-volatile storage media, such as (but not necessarily) a relational database. Alternatively, or additionally, the database 22 may further comprise or consist of a cloud based solution, wherein storage and/or processing of data is performed in a cloud.

According to particular embodiments of the disclosed method/system, the control unit 20 used for localizing the handheld analytical devices 10 is shared with (the same computerized machine as) the functionality to manage the handheld analytical devices 10 in the point of care system 1 such as the cobas IT 1000 application by Roche Diagnostics, functionality including but not limited to: access rights-/certificates-/operator- management, result validation and visualization, inventory management, remote configuration and control, etc.

A connection to a hospital information system 50 of particular embodiments is illustrated in figure 1 with a dashed line, the connection to a hospital information system 50 (or other information system containing patient and/or staff related data) being particularly advantageous for retrieving information related to patients, in particular the location of a patient within the point of care environment, e.g. a hospital or clinician's office.

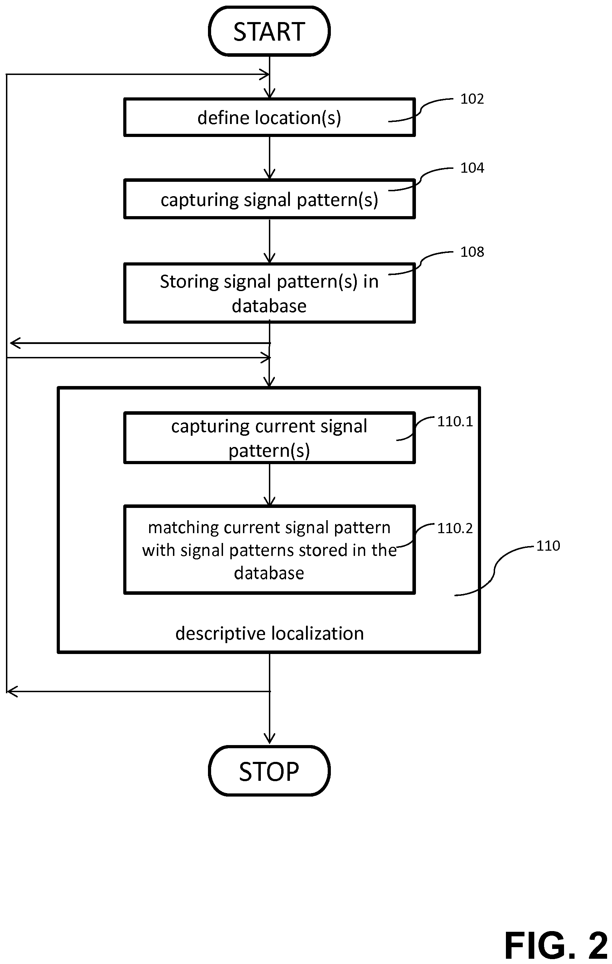

Turning now to figure 2, a first embodiment of the disclosed method shall be described.

In a step 102, a location identifier 30 is associated with one or more of the plurality of locations within the point of care environment. The location identifier 30 is either a description of the location such as "Room 1", "nurse's desk", "Emergency room", "oncology ward", "cardiology west wing", "Hospital X, 1^{st} floor". Alternatively, or additionally, the location identifier is a numerical number associated with the description of the location. The granularity of the definition of location within the point of care environment is chosen by the level of accuracy of the localization that needs to be achieved and/or based on the efforts/capabilities appropriate given the particular use case. Also, according to embodiments of the invention, the granularity of the definition of location identifiers 30 may be increased iteratively as more and more locations are defined more precisely with the system 1 already in use. It shall be noted that there are practical and/or technical limitations to the granularity of the definition of location identifiers 30. Commonly a localization more precise than room level is technically challenging/expensive with little or no practical benefit for the user. Therefore, often a hierarchical definition of locations is preferred, such as room > ward section> ward floor> building > site.

Thereafter the location identifier 30 associated with one or more locations is stored into the database 22.

In a following step 104, signal patterns are captured using signal receiver(s) of the plurality of the handheld analytical devices 10. In particular embodiments herein disclosed, the signal receiver(s) used to capture the signal patterns of the surroundings are signal receivers already part of the handheld analytical devices 10 so that no dedicated hardware needs to be deployed/the handheld analytical devices 10 need not be modified. As such the handheld analytical devices 10 may be off-the-shelf/known POC devices with only software configuration to implement the method herein disclosed. The signal pattern captured by the signal receiver(s) of the handheld analytical devices 10 is also referred to as signal fingerprint and comprises a snapshot of signals detected at that moment by the signal receiver(s). In addition, off-the-shelf portable computing devices (such as smartphones) may also be used to complement the teaching /capture of signal patterns across the point of care environment. According to various embodiments of the disclosed method/system, the signals patterns captured by the handheld analytical devices 10 comprises one or more from the list comprising (but not limited to):
- Radio signal pattern(s), wherein one or more of the plurality of the handheld analytical devices 10 comprises signal receiver(s) configured to capture radio signal patterns, such as a Wi-Fi, Bluetooth, Ultra Wide Band UWB or cellular radio signal pattern and optionally wherein the point of care environment further comprising a plurality of radio signal transmitters. It shall be noted that the radio signal pattern(s) captured is the combination of all radio signals (or e.g. a subset thereof of a certain frequency band) detectable in the vicinity of the handheld analytical device 10 and may comprise radio signals emitted by several radios signal emitters.
   Sensors for capturing radio signal patterns are known in the art and are commonly integrated already in handheld analytical devices 10. Alternatively and/or additionally, the handheld analytical devices 10 may be retrofitted with such sensors (such as a usb connected radio transceiver).
   Similarly, according to various embodiments herein disclosed, the source of the radio signals is from radio transmitters already part of a known point of care environment, i.e. radio signal transmitters are shared for the purpose of connecting the handheld analytical devices 10 to a POC IT application and the control unit 20. Alternatively, or additionally, dedicated radio signal transmitters are provided for the purpose to support the localization of the handheld analytical devices 10. For an approximate localization, collected Wi-Fi signal patterns can additionally be evaluated by third party localization services.
- Sound signal patterns, wherein one or more of the plurality of the handheld analytical devices 10 comprises signal receiver(s) configured to capture sound signal patterns, such as background noise or audible clues indicative of a location within the point of care environment. Sensors for capturing sound signal patterns are known in the art and are sometimes integrated already in handheld analytical devices 10, such as a microphone. Alternatively and/or additionally, the handheld analytical devices 10 may be retrofitted with such sensors (such as a usb connected microphone).
   According to various embodiments herein disclosed, the source of the sound signals may be characteristic sounds present anyhow in a point of care environment, such as noise from particular instruments and installations, voices of personnel, etc. Alternatively, or additionally, dedicated sound transmitters are provided for the purpose to support the localization of the handheld analytical devices 10, such as sound generators of a frequency not detectable by humans.
- Light signal pattern, wherein one or more of the plurality of the handheld analytical devices 10 comprises signal receiver(s) configured to capture light signal patterns, such as light signal patterns being indicative of whether the handheld analytical device 10 is located in an enclosure such as a drawer, cupboard or the like. Sensors for capturing light signal patterns are known in the art and are sometimes integrated already in handheld analytical devices 10, such as a photodiode or a camera. According to various embodiments herein disclosed, the source of the light signals may be light sources present anyhow in a point of care environment, such as lamps, computer screens, sunlight, etc. These light signal patterns are used in particular to detect relative location of the handheld analytical device 10, such as on a table (when light is detected) or in a drawer/closed cupboard, when no light is detected. Alternatively, or additionally, dedicated light transmitters are provided for the purpose to support the localization of the handheld analytical devices 10, such as IR light emitters (beacons).
- Atmospheric pressure, wherein one or more of the plurality of the handheld analytical devices 10 comprises signal receiver(s) configured to capture atmospheric pressure, the captured atmospheric pressure being indicative of an elevation of the handheld analytical device 10 such as a floor within the point of care environment. Sensors for capturing light signal patterns are known in the art and are sometimes integrated already in handheld analytical devices 10. Alternatively, or additionally, the handheld analytical devices 10 may be retrofitted with such sensors (such as a usb connected altimeter).
- Visual pattern such as a photo or video recording, wherein one or more of the plurality of the handheld analytical devices 10 comprises signal receiver(s) configured to capture a visual pattern, the visual pattern such as a QR or barcode identifying or being indicative of a location within the point of care environment. Sensors for capturing a visual pattern are known in the art and are sometimes integrated already in handheld analytical devices 10. Alternatively, and/or additionally, the handheld analytical devices 10 may be retrofitted with such sensors (such as a usb connected camera). The visual patterns captured are either patterns already part of the point of care environment or patterns specifically placed to facilitate the localization of the handheld analytical devices 10, such as QR codes placed at various locations.
- Motion/orientation patterns, wherein one or more of the plurality of the handheld analytical devices 10 comprises motion/orientation sensors (e.g. gyroscope), the motion/orientation patterns being indicative of whether the handheld analytical device 10 is currently carried by someone or if it is idle somewhere (e.g. on a table, in a drawer). Furthermore, an upright orientation may be indicative that the handheld analytical device 10 is located in a docking station.
- Environmental pattern such as temperature & relative humidity, the environmental pattern being indicative of whether the handheld analytical device 10 is in a room with a controlled environment (such as ICU).
- Magnetic field, wherein the handheld analytical devices 10 comprise (or are connected to a portable computing device comprising) magnetic sensors, wherein the magnetic field is indicative of a geographical location of the handheld analytical devices 10 based on state-of-the art technology of localization based on characteristic magnetic fields of buildings, respectively unique magnetic landscapes produced by the Earth's magnetic field that interacts with steel and other materials found in structures of buildings.
- Olfactory pattern, wherein one or more of the plurality of the handheld analytical devices 10 comprises signal receiver(s) configured to capture an olfactory pattern such as a characteristic olfactory patterns at medical areas (e.g. dentist), meeting areas (e.g. coffee or lunch rooms).

In a following step 108, the signal patterns captured by the plurality of handheld analytical devices 10 are stored in the database 22, each signal pattern being associated with the location identifier 30 where the signal pattern has been captured. According to embodiments of the disclosed method/system, the association of the signal pattern with the location identifier 30 is performed either automatically based on a previous association of a priori information of the location of the captured signal pattern or manually by an operator of the handheld analytical device or an operator of the control unit 22, in particular via a user interface 24 connected thereto.

As illustrated on figure 2, according to embodiments herein disclosed, steps 102 (defining of locations); step 104 (capturing signal patterns) and step 108 are carried out iteratively and incrementally, i.e. the definition of locations, the capture of the corresponding signal pattern(s) and their storage in the database must not be all done at once but can be performed one location at a time, the database being populated incrementally over time with each iteration - e.g. during daily routine use of the handheld analytical devices 10. To be noted that for each location teaching iteration (steps 102, 104, 108) the same or different handheld analytical devices 10 may be employed, hence enabling a collaborative buildup of the database 22 of signal patterns.

After at least one teaching iteration (definition of at least one location identifier 30 associated with a location of the point of care environment respectively the capture and storage of the corresponding signal pattern), the steps related to descriptive localization 110 follow.

In a first substep 110.1 of the descriptive localization 110, the current signal pattern is captured using signal receiver(s) of a handheld analytical device 10 to be localized. The term '**current**' as used herein shall refer not only to an absolutely real-time determination of the actual signal pattern around a handheld analytical device 10 but also to a relatively current signal pattern, namely the latest signal pattern captured by a handheld analytical device 10. As technological limitations (such as connectivity delays) may prevent an absolute real-time capture of current signal patterns, both real-time as well as latest captured signal patterns are commonly referred to here as "current" signal patterns. Therefore, the term current shall be interpreted in a broad sense to cover the most up-to-date signal pattern captured by the particular handheld analytical device 10 which is then used for determining the current location of the handheld analytical device 10. Accordingly, the "current location" of the handheld analytical device 10 determined corresponds to the moment in time the "current signal pattern" has been captured and therefore may deviate from the absolute real-time location of the handheld analytical device 10.

According to embodiments of the disclosed method/system, capturing signal patterns and/or the current signal pattern by the handheld analytical device(s) 10 is:
- triggered by user action(s) carried out on the respective handheld analytical device(s) 10 (please refer to paragraphs below for a detailed description of such user actions); and/or
- triggered on a recurring basis defined by a signal capture schedule, e.g. every minute, hour, at the start/end of a work shift; and/or
- self-triggered by the handheld analytical device 10 depending on a characteristic status e.g. low battery or upcoming issue which disconnects from network; and/or
- manually triggered by an operator of the respective handheld analytical device(s) 10; and/or
- triggered by the control unit 20 when the respective handheld analytical device(s) 10 is to be localized, i.e. remotely triggered by the control unit 20 (the control unit 20 queries the handheld analytical device 10 for its current signal pattern).

In following substep 110.2, the control unit 20 performs a comparison of the current signal pattern with the signal patterns stored in the database 22 to identify a signal pattern matching the current signal pattern. The term "match"/" matching" - with reference to a signal pattern matching another - as used herein comprises not only an absolutely identical signal patterns but also covers approximately respectively partially matching signal patterns. In other words, two signal patterns are considered to match according to embodiments disclosed herein if any degree of overlap there-between can be identified. The degree of overlap between matching signal patterns varies by case to case and is selected high enough such as to avoid false matches on one hand, but on the other hand low enough to ensure that subsequent signal patterns measurements in the approximately the same location are identified despite these not being 100% identical. For this purpose, known state-of-the-art algorithms can be applied such as algorithms based on the 'k-nearest neighbor (kNN)'.

Furthermore, if radio signal patterns captured using a radio signal transmitter of a handheld analytical device 10 in one part of a room differ from radio signal patterns captured in other parts of the same room, the degree of overlap between two signal patterns considered to match is selected such as to ensure that signal patterns captured indeed in the same room are identified as matching, yet radio signal patterns captured outside the room are not falsely identified as matching.

Following the matching of the current signal pattern with the signal pattern(s) stored in the database 22, the control unit 20 provides a descriptive localization of the handheld analytical device 10, the descriptive localization comprising an indication of a position of the handheld analytical device 10 to be localized relative to one or more of the plurality of locations within the point of care environment associated with one or more signal pattern(s) stored in the database 22 matching the current signal pattern. According to embodiments disclosed herein, the descriptive localization of the handheld analytical device 10 is provided on a user interface 24 connected to or comprised by the control unit 20. Alternatively, or additionally, the descriptive localization of the handheld analytical device 10 is provided on a portable computing device such as smartphone, tablet or even on a screen of a different handheld analytical device 10 than the handheld analytical device 10 to be localized.

The term '**descriptive localization**' shall be used in the context of the present application to refer to any form of indication of the location of a handheld analytical device 10 within the point of care environment, wherein the location is provided in the form of a sentence (as text or spoken) descriptive of the location of a handheld analytical device 10 relative to a defined location as opposed to an absolute localization e.g. on a map. The localization according to the disclosed method/system is called descriptive to distinguish over absolute or coordinate based localization known in the art. Soft localization by means of descriptive localization is advantageous in a point of care environment, as the descriptive localization is correlated to the daily work of the POC operators, e.g. "the handheld analytical device 10 you are looking for is where you mostly perform QC measurements"; "the handheld analytical device 10 you are looking for is in the vicinity of the bedside of patient G. Smith", "the handheld analytical device 10 you are looking for is in the office of Dr. Smith"; "the handheld analytical device 10 you are looking for is around the docking station, probably in a closed drawer (based on complete lack of light around the device)", etc.

According to further embodiments of the disclosed method/system, an indicator of probability is associated with the descriptive localization of a position of the handheld analytical device 10, in particular if the current signal pattern matches only partially with signal pattern(s) stored in the database 22. In such as case, the descriptive localization of the handheld analytical device 10 may take the form "the handheld analytical device 10 you are searching is located probably/most probably/maybe in room 1". Alternatively, or additionally, the probability may be brought into a spatial approximation, e.g. the handheld analytical device 10 you are searching is located 5 to 10 meters from the docking station in room 1", "...located within or close to room", "...located within oncology ward/west wing", etc.

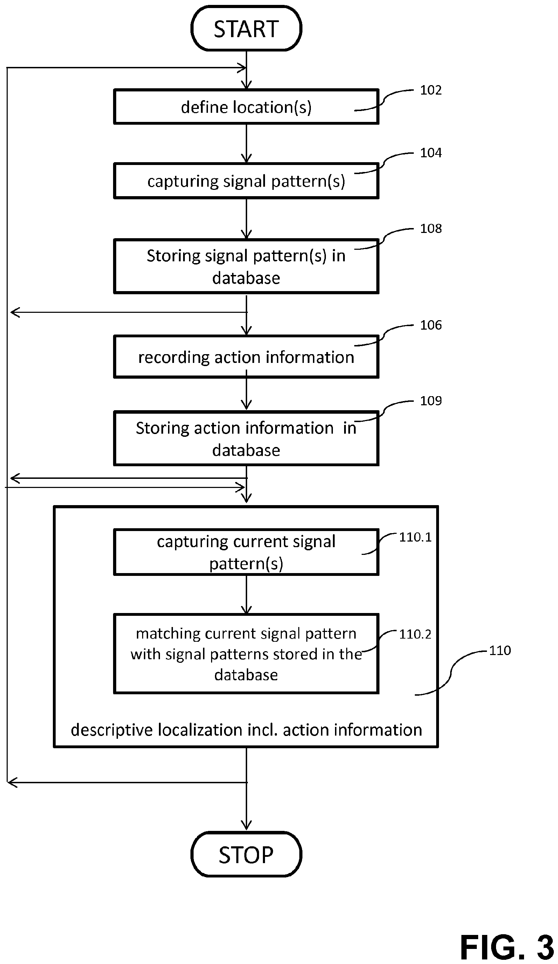

Turning now to figure 3, further embodiments of the disclosed method shall be described, wherein the descriptive localization of the handheld analytical devices 10 is enriched by providing action information (as part of the descriptive localization) related to user action(s) historically carried out at the current location of the searched handheld analytical device 10.

According to these embodiments, the step 102 of defining locations further comprises storing of association(s) between one or more user action(s) and one or more of the plurality of locations within the point of care environment into the database 22. As illustrated on figure 2, the method further comprises step 106 of recording action information upon a user action being carried out on one or more of the handheld analytical device(s) 10. The action information is indicative of the user action being carried out, such as a description of the action, e.g. "quality control", "sample measurement", etc. The action information is either input manually by an operator of the handheld analytical device 10 directly using the handheld analytical device 10 or via the user interface 24 of the control unit 20. Alternatively, or additionally, the action information is retrieved from a laboratory or hospital information system communicatively connected to the control unit 20. According to embodiments of the disclosed method/system, the action information also comprises a reference to the handheld analytical device 10 on which the user action has been carried out and/or an operator that carried out the user action.

In a step 109, all action information captured by the plurality of handheld analytical devices 10 is stored into the database 22, each action information being associated with the location identifier 30 associated with the location where the user action was carried out.

According to the embodiments illustrated on figure 3, in order to enrich the descriptive localization of the handheld analytical device 10 to be localized, the descriptive localization further comprises the action information associated with one or more of the plurality of locations within the point of care environment associated with one or more signal pattern(s) stored in the database 22 matching the current signal pattern. For example, the descriptive localization could be phrased as "The lost handheld device is in or close to the room where devices x,y and z have been charged in the past".

It is apparent that in this way, localization of a "lost" handheld analytical device 10 is further aided by the actions carried out by other handheld analytical devices 10 in the same point of care environment.

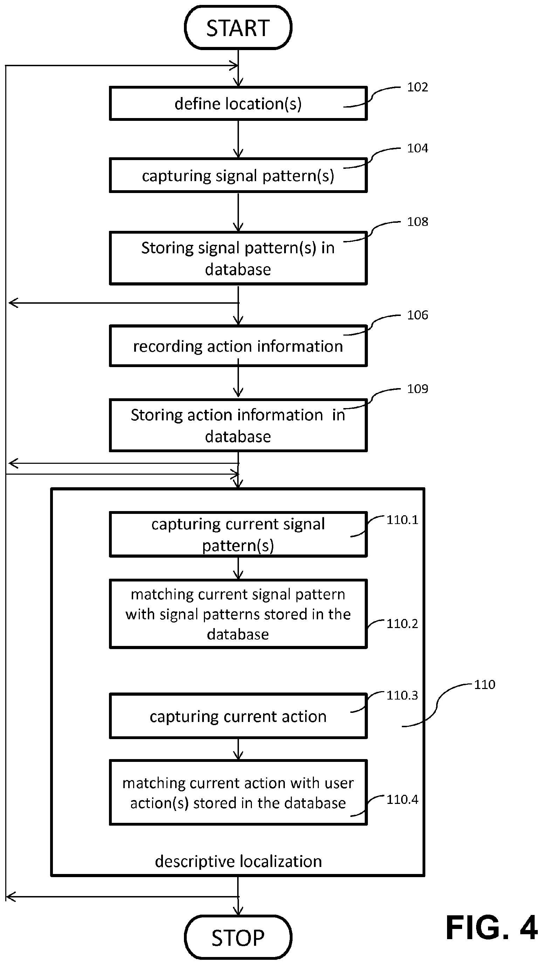

Figure 4 depicts a flowchart of further embodiments of the disclosed method, wherein the localization is further making use of the currently performed action on the handheld analytical device 10. In order to achieve this, the step 110 of descriptive localization further comprises substeps 110.3 of capturing the most recent action respectively substep 110.4 of matching most recent action with user action(s) in the database 22.

In substep 110.3, the most recent action is captured, the most recent user action being indicative of user actions most recently carried out with or on the handheld analytical device 10 to be localized. Nevertheless, the term 'most recent user action' also comprises a user action currently being carried out. A descriptive localization based on a current user action /most recent action is advantageous when a user other than the current user of the handheld analytical device 10 is searching for that particular handheld analytical device 10. For example, a nurse needs to perform an analysis of a patient sample while the handheld analytical device 10 needed for this procedure is currently being serviced (or has most recently been serviced) by service personnel. Additionally, or alternatively, the descriptive localization comprises and indication of the logged-in user of the handheld analytical device 10 corresponding to the current user action /most recent action.

In a following substep 110.4, the control unit 20 performs a comparison of the most recent action with user actions stored in the database 22 to identify a user action matching the most recent action. Thereafter - based on this comparison, the control unit 22 provides a descriptive localization of the handheld analytical device 10 comprising an indication of the position of the handheld analytical device 10 to be localized relative to one or more of the plurality of locations within the point of care environment associated with one or more user action(s) stored in the database 22 matching the most recent action. Following up on the example of the preceding paragraph, the nurse searching for the handheld analytical device 10 which has just been serviced by service personnel - the servicing user action being associated with the service station - would receive a descriptive localization as "the handheld analytical device 10 you are searching has just been serviced and is therefore at the service station".

The user actions which are captured (either to be stored in the database 22 or for matching with previously stored user actions) comprise one or more from the list comprising (but not limited to):
- docking/charging of the handheld analytical device 10 into a docking station, wherein the action information comprises an indication of the relative location of the docking/charging station with respect to one or more of the plurality of locations of the point of care environment. According to particular embodiments disclosed herein, different types of handheld analytical devices 10 are associated with different locations for docking stations. Furthermore, action information may further comprise an indication of charging status and/or expected completion of charging of the handheld analytical device 10;
- performing of a quality control QC procedure of the handheld analytical device 10, wherein the action information comprises one or more of the plurality of locations of the point of care environment where quality control QC procedure of the handheld analytical device 10 is known to be carried out. According to particular embodiments disclosed herein, different types of handheld analytical devices 10 are associated with different locations for performing of quality control QC. Furthermore, action information may further comprise an indication of expected completion time of the quality control QC procedure;
- reagent and/or consumables (e.g. test strips) lot scanning procedure using the handheld analytical device 10, wherein the action information comprises one or more of the plurality of locations of the point of care environment where reagent lot scanning procedure is known to be carried out. According to particular embodiments disclosed herein, different types of handheld analytical devices 10 are associated with different locations for reagent lot scanning and/or different types of reagents are associated with different locations for reagent lot scanning;
- servicing procedure of the handheld analytical device 10, wherein the action information comprises one or more of the plurality of locations of the point of care environment where servicing procedures of the respective handheld analytical device 10 are known to be carried out. According to particular embodiments disclosed herein, different types of handheld analytical devices 10 are associated with different locations for servicing;
- analytical testing of a biological sample of a patient, wherein the action information comprises the location within the point of care environment corresponding to the testing of the respective patient, optionally wherein the location corresponding to the testing of the respective patient is retrieved from a hospital information system 50 communicatively connected to the control unit 20;
- identification of an operator of the handheld analytical device 10, wherein the action information comprises a list of locations within the point of care environment where the operator is assigned - such as a hospital ward, optionally wherein the location where the operator is assigned is retrieved from a hospital information system communicatively connected to the control unit 20.

According to further embodiments of the disclosed method/system, the method further comprises one or more of the following steps:
- Updating the signal pattern associated with a location identifier 30. If the signal environment of a location changes (e.g. a new radio signal transmitter - such as a Wi-Fi access point - is placed), the signal pattern in the database 22 associated with that location is no longer up-to-date and is updated. The update might be triggered either automatically (self-monitoring/self-check) by the control unit 20 identifying that the signal pattern in the database 22 associated with that location is no longer up-to-date or manually by an operator or service personnel being aware of the change in the signal environment;
- Updating the location identifier 30 associated with a signal pattern. If a signal emitter (e.g. a radio signal transmitter - such as a Wi-Fi access point) is relocated to a new location within the point of care environment, then the location identifier associated with the corresponding signal pattern is no longer up-to-date and is updated to correspond to the new location of the respective signal pattern. The update of the location identifier 30 associated with a signal pattern might be triggered either automatically by the control unit 20 identifying that the location identifier associated with the corresponding signal pattern is no longer up-to-date or manually by an operator or service personnel being aware of the change in the signal environment;
- Updating the association(s) between one or more user action(s) and one or more of the plurality of locations within the point of care environment. If the location where a user action is changed within the point of care environment (such as relocation of patient(s), operator(s), equipment, etc.), the location identifier associated with the corresponding user action is no longer up-to-date and is updated to correspond to the new location of the respective user action.

According to further embodiments of the disclosed method/system, an indicator of probability is associated with the descriptive localization of a position of the handheld analytical device 10 in situations comprising one or more from the list comprising (but not consisting of):
- Two or more of the signal pattern(s) stored in the database 22 match (at least partially) the current signal pattern.
- Two or more of the plurality of locations within the point of care environment are associated with user action(s) stored in the database 22 matching the most recent action, such as:
   ∘ two or more locations are associated with the docking/charging user action;
   ∘ two or more locations are associated with performing quality control QC procedures of the handheld analytical device 10;
   ∘ two or more locations are associated with performing reagent lot scanning procedures of the handheld analytical device 10;
   ∘ two or more locations are associated with performing servicing procedures of the handheld analytical device 10;
   ∘ two or more locations are associated with performing analytical testing of a biological sample of a patient;
   ∘ two or more locations are assigned to an operator who performed an identification of an operator of the handheld analytical device 10.

In any of the above cases, the probability associated with the descriptive localization may be a numerical and/or descriptive indication of probability. For example: "the handheld analytical device 10 you are looking for has been most recently used to measure a blood sample of patient Smith. Therefore, this handheld analytical device 10 is either in room 1 with the hospital bed of patient Smith or in treatment room 2 where Dr. Johnson has his office" and/or "the handheld analytical device 10 you are looking for has been most recently used to measure a blood sample of patient Smith. Therefore, there is a 50% probability that this handheld analytical device 10 is in room 1 with the hospital bed of patient Smith a 50% probability that it is in treatment room 2 where Dr. Johnson has his office". Alternatively, or additionally, a more user-friendly descriptive localization is provided, such as "the handheld analytical device 10 you are looking for las been used to measure a blood sample of patient Smith. Therefore, this handheld analytical device 10 probably in room 1 or in treatment room 2".

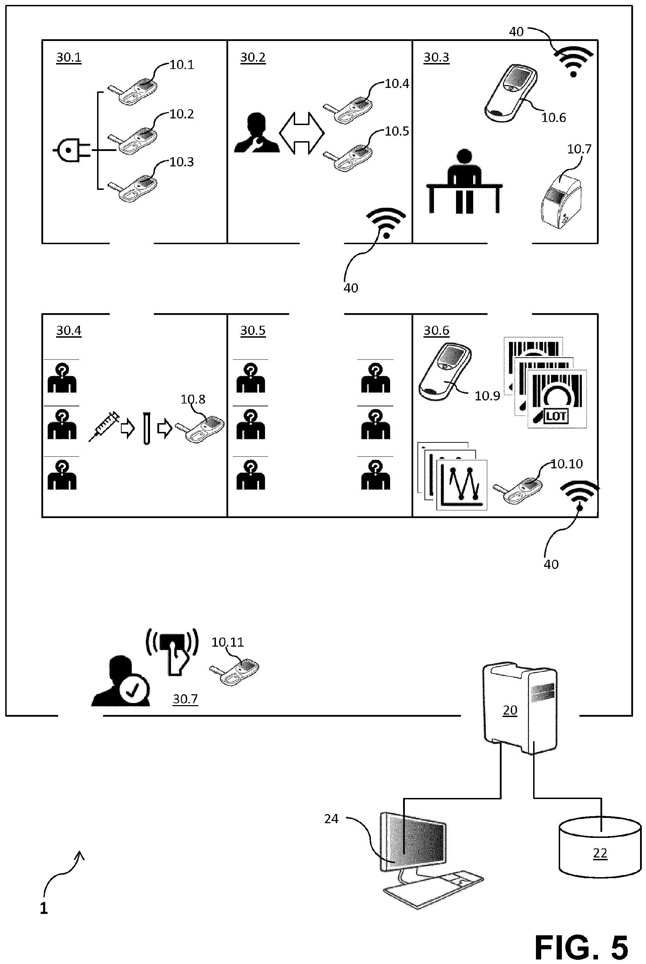

Figure 5 a schematic diagram of a particular example of a point of care system 1 according to the present disclosure as deployed in a point of care environment. Figure 5 illustrates a hospital 1 (as point of care environment) comprising several locations 30.1 - 30.7 as follows:
- A charging location 30.1 comprising a docking/charging station where several handheld analytical devices 10 are brought to be charged;
- A service location 30.2, where handheld analytical devices 10 are serviced;
- A clinician's office 30.3 where a clinician uses handheld analytical devices 10 for analytical testing of a biological sample of patients;
- Hospital rooms with patient beds 30.4, respectively 30.5, where nurses perform analytical testing of a biological sample of patients;
- A storage room 30.6 where reagents are stored and retrieved for use followed by lot scanning using the handheld analytical device 10;
- An entry location 30.7, where hospital personnel (operators) identify themselves for using the handheld analytical devices 10.

As illustrated on figure 5, locations 30.2, 30.3 and 30.6 comprise wireless network access points emitting radio signals which may be captured in a step 104 respectively 110.1 of the method as described in preceding paragraphs.

Assuming teaching of the signal patterns has been performed for each of the locations 30.1-30.7 shown on figure 5, the descriptive localization of the handheld analytical devices 10.1-10.11 of figure 5 could for example be provided by the control unit 20 as follows:
- "Handheld analytical devices 10.1 - 10.3 are in charging location 30.1 - (based on the most current user action of docking)". Optionally the descriptive localization may further comprise "Handheld analytical device 10.1 is ready for use in 10 minutes based on its last known battery level";
- "Handheld analytical devices 10.4 and 10.5 are in the service location 30.2 (based on the current radio signal pattern matching the radio signal pattern of the service location 30.2)";
- "Handheld analytical devices 10.6 and 10.7 are in clinician's office 30.3 (based on the current radio signal pattern matching the radio signal pattern of clinician's office 30.3)";
- "Handheld analytical device 10.8 is most probably in hospital room 30.4 (based on the most recent action being analytical testing of a biological sample of Mr. Smith who is hospitalized in a bed in room 30.4)";
- "Handheld analytical devices 10.9 and 10.10 are in the room 30.6 where QC and lot reagent scanning is usually performed (based on the most recent action and also based on action information associated with room 30.6)";
- "Handheld analytical device 10.11 is most probably around the entry location 30.7 (based on the most recent action being identification by Dr. Williams)".

In the following, a particular implementation of the disclosed method/system is described.
1. The handheld analytical devices 10 are installed by the field system engineers of the vendor. As part of this process a location identifier 30 corresponding to one or more of a plurality of locations within the point of care environment is associated with each stationary element (such as docking/charging stations).
   The location ID 30 comprises the following:
   a. dock name (e.g. dock "blue", "green")
   b. site (e.g. University Hospital Zürich)
   c. ward (e.g. Oncology, Cardiology, ICU)
   d. ward section (e.g. West wing, east wing, patient bays)
   e. room (e.g. nurse's desk, storage, office Dr. Smith)
      Example used in the following "Dock name: Green; room: nurse's desk; ward section: patient bays ward: oncology"
2. Each time a device 10 is docked, a (radio) signal pattern is captured and transmitted with the location ID of the dock in norder to iteratively and collaboratively (by each device 10) self-teach & continuously monitor the signal environment (e.g. changes are detected, (re)moved docks are detected).
3. Localization of the handheld analytical devices 10.
   As it can be seen, a certain level of descriptive localization is already possible already after merely self-teaching the (radio) signal patterns of a single docking station, self-teaching triggered automatically each time a handheld analytical device 10 is docked. E.g.:
   a. Device is in room of dock green (room: "nurse's desk", section "patient bays" ward: oncology)
   b. Device is in or close to room of dock green (room: "nurse's desk", section "patient bays" ward: oncology)
   c. Device is in a neighbor room of dock green (room: "nurse's desk", section "patient bays" ward: oncology)
   d. Device is some rooms away of dock green within ward oncology
4. Other docks maybe installed in other rooms and further localizations can be done. By self-teaching of the radio signal patterns corresponding to further docking station(s), the localization capabilities are extended. E.g.:
   Device is between rooms of docks green (room ....) and red (room ..., ward ...).
5. As users will not place the docks to get the best localization results but most probably will place them together somewhere, manual teaching may be needed for some locations. In such cases the user takes the teaching device (handheld analytical devices 10 or mobile device) and teaches manually by walking around the point of care environment. With this option, users can decide which areas (locations) are covered in which granularity for their localization needs.
6. The descriptive localization is thus based on the closest location IDs 30 and a descriptive distance to its location based on two parameters:
   a. The location: within ranges: from "room- ward section - ward" (optionally plus dock name)"
   b. and a distance: within, within or close to, in a neighbor room, a floor away.

It will be understood that the above are examples to illustrate the present invention and that many variations could be adopted based on the specific structure hereinbefore described without departing from the scope as defined in the claims.

Further disclosed and proposed is a computer program product including computer-executable instructions for performing the disclosed method in one or more of the embodiments enclosed herein when the program is executed on a computer or computer network. Specifically, the computer program may be stored on a computer-readable data carrier or a server computer. Thus, specifically, one, more than one or even all of method steps as indicated above may be performed by using a computer or a computer network, preferably by using a computer program.

As used herein, a computer program product refers to the program as a tradable product. The product may generally exist in any format, such as in a paper format, or on a computer-readable data carrier on premise or located at a remote location. Specifically, the computer program product may be distributed over a data network (such as a cloud environment). Furthermore, not only the computer program product, but also the execution hardware may be located on premise or in a cloud environment.

Further disclosed and proposed is a computer-readable medium comprising instructions which, when executed by a computer system, cause a laboratory system to perform the method according to one or more of the embodiments disclosed herein.

Further disclosed and proposed is a modulated data signal comprising instructions which, when executed by a computer system, cause a laboratory system to perform the method according to one or more of the embodiments disclosed herein.

Referring to the computer-implemented aspects of the disclosed method, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

**REFERENCE LIST:**

| | |
|---|---|
| point of care system | 1 |
| handheld analytical device(s) | 10, 10.1-10.m |
| control unit | 20 |
| database | 22 |
| user interface | 24 |
| location identifier | 30, 30.1-30.n |
| communication network | 40 |
| hospital information system | 50 |
| defining locations | step 102 |
| capturing signal patterns | step 104 |
| recording of action information | step 106 |
| storage of signal patterns | step 108 |
| storage of action information | step 109 |
| descriptive localization | step 110 |
| capturing current signal pattern | substep 110.1 |
| matching current signal pattern with signal patterns in the database | substep 110.2 |
| capturing most recent action | substep 110.3 |
| matching most recent action with user action(s) in the database | substep 110.4 |

## Claims

1. A computer implemented method for localizing handheld analytical devices (10) in a point of care environment, the point of care environment comprising a plurality of handheld analytical devices (10) for analyzing biological samples communicatively connected to a control unit (20), the method comprising:
- associating a location identifier (30) corresponding to one or more of a plurality of locations within the point of care environment and storing said location identifier (30) into a database (22) communicatively connected to or comprised by the control unit (20);
- at each of the plurality of locations, capturing signal patterns using signal receiver(s) of the plurality of the handheld analytical devices (10);
- storing all signal patterns captured by the plurality of handheld analytical devices (10) in a database (22), each signal pattern being associated with the location identifier (30) where the signal pattern has been captured;
- capturing a current signal pattern using signal receiver(s) of a handheld analytical device (10) to be localized;
- the control unit (20) providing a descriptive localization, the descriptive localization comprising an indication of a position of the handheld analytical device (10) to be localized relative to one or more of the plurality of locations within the point of care environment associated with one or more signal pattern(s) stored in the database (22) matching the current signal pattern.

2. The method according to claim 1, wherein capturing signal patterns and/or the current signal pattern by the handheld analytical device(s) (10) is:
- triggered by user action(s) carried out on the respective handheld analytical device(s) (10); and/or
- triggered on a recurring basis based on a signal capture schedule; and/or
- manually triggered by an operator of the respective handheld analytical device(s) (10); and/or
- self-triggered by the handheld analytical device(s) (10) due to a characteristic status such as 'low battery', 'low network signal', upcoming issue'; and/or
- triggered by the control unit (20) when the respective handheld analytical device(s) (10) is to be localized.

3. The method according to claim 1 or 2, the method further comprising:
- storing into the database (22) an association(s) between one or more user action(s) and one or more of the plurality of locations within the point of care environment;
- recording action information upon a user action being carried out on one or more of the handheld analytical device(s) (10), the action information being indicative of the user action being carried out;
- storing all action information captured by the plurality of handheld analytical devices (10) in the database (22), each action information being associated with the location identifier (30) associated with the location where the user action was carried out,
wherein the descriptive localization of the handheld analytical device (10) to be localized further comprises the action information associated with one or more of the plurality of locations within the point of care environment associated with one or more signal pattern(s) stored in the database (22) matching the current signal pattern.

4. The method according to claim 3, the method further comprising
capturing a most recent action carried out by the handheld analytical device (10) to be localized,
wherein the descriptive localization comprises an indication of a position of the handheld analytical device (10) to be localized relative to one or more of the plurality of locations within the point of care environment associated with one or more user action(s) stored in the database (22) matching the most recent action.

5. The method according to claims 3 or 4, wherein the user actions comprise one or more from the list comprising:
- docking/charging of the handheld analytical device (10) into a docking station, wherein the action information comprises an indication of the relative location of the docking/charging station with respect to one or more of the plurality of locations of the point of care environment;
- performing of a quality control QC procedure of the handheld analytical device (10), wherein the action information comprises one or more of the plurality of locations of the point of care environment where quality control QC procedure of the handheld analytical device (10) is known to be carried out;
- reagent and/or consumable lot scanning procedure using the handheld analytical device (10), wherein the action information comprises one or more of the plurality of locations of the point of care environment where reagent lot scanning procedure is known to be carried out;
- servicing procedure of the handheld analytical device (10), wherein the action information comprises one or more of the plurality of locations of the point of care environment where servicing procedures of the respective handheld analytical device (10) are known to be carried out;
- analytical testing of a biological sample of a patient, wherein the action information comprises the location within the point of care environment corresponding to the testing of the respective patient, optionally wherein the location corresponding to the testing of the respective patient is retrieved from a hospital information system (50) communicatively connected to the control unit (20);
- identification of an operator of the handheld analytical device (10), wherein the action information comprises a list of location(s) within the point of care environment where the operator is assigned - such as a hospital ward, optionally wherein the location where the operator is assigned is retrieved from a hospital information system communicatively connected to the control unit (20).

6. The method according to one of the preceding claims 1 to 5, wherein the signals patterns captured by the handheld analytical devices (10) comprises one or more from the list comprising:
- radio signal pattern(s), wherein one or more of the plurality of the handheld analytical devices (10) comprises signal receiver(s) configured to capture radio signal patterns, such as a Wi-Fi, Bluetooth or cellular radio signal pattern and optionally wherein the point of care environment further comprises a plurality of radio signal transmitters;
- sound signal patterns, wherein one or more of the plurality of the handheld analytical devices (10) comprises signal receiver(s) configured to capture sound signal patterns, such as background noise or audible clues indicative of a location within the point of care environment;
- light signal pattern, wherein one or more of the plurality of the handheld analytical devices (10) comprises signal receiver(s) configured to capture light signal patterns, such as light signal patterns being indicative of whether the handheld analytical device (10) is located in an enclosure such as a drawer, cupboard or the like;
- atmospheric pressure, wherein one or more of the plurality of the handheld analytical devices (10) comprises signal receiver(s) configured to capture atmospheric pressure, the captured atmospheric pressure being indicative of an elevation of the handheld analytical device (10) such as a floor within the point of care environment;
- visual pattern such as a photo or video recording, wherein one or more of the plurality of the handheld analytical devices (10) comprises signal receiver(s) configured to capture a visual pattern, the visual pattern such as a QR or barcode identifying or being indicative of a location within the point of care environment;
- motion/orientation pattern, wherein one or more of the plurality of the handheld analytical devices (10) comprises motion/orientation sensors, the motion/orientation pattern being indicative of whether the handheld analytical device (10) is currently in motion or if it is idle;
- environmental pattern such as temperature & relative humidity, the environmental pattern being indicative of whether the handheld analytical device (10) is in a room with a controlled environment;
- magnetic field, wherein one or more of the plurality of the handheld analytical devices (10) comprise a magnetic sensor, wherein the magnetic field is indicative of a geographical location of the handheld analytical devices (10);
- olfactory pattern, wherein one or more of the plurality of the handheld analytical devices (10) comprises signal receiver(s) configured to capture an olfactory pattern such as a characteristic olfactory pattern.

7. The method according to one of the preceding claims 1 to 6, wherein the method further comprises:
- updating the signal pattern associated with a location identifier (30);
- updating the location identifier (30) associated with a signal pattern;
- updating the association(s) between one or more user action(s) and one or more of the plurality of locations within the point of care environment.

8. The method according to one of the preceding claims 1 to 7, wherein the method further comprises associating an indicator of probability to the descriptive localization of a position of the handheld analytical device (10) to be localized if:
- two or more of the signal pattern(s) stored in the database (22) match the current signal pattern; and/or
- two or more of the plurality of locations within the point of care environment are associated with user action(s) stored in the database (22) matching the most recent action.

9. Point of care system (1) for analyzing biological samples comprising:
- a plurality of handheld analytical devices (10) for analyzing biological samples;
- a control unit (20) communicatively connected to the plurality of handheld analytical devices (10) by means of a communication network (40);
- a database (22) communicatively connected to or comprised by the control unit (20),
wherein the point of care system is configured such as to carry out one of the methods according to the preceding claims.

10. A computer program product comprising instructions which, when executed by a computer system, cause a point of care system to perform the steps of any one of the methods according to claims 1 through 8.
